Europäisches Patentamt

European Patent Office

Office européen des brevets

(11) Publication number: **0 209 370**
**A2**

## EUROPEAN PATENT APPLICATION

(21) Application number: **86305457.3**

(51) Int. Cl.⁴: **C 12 N 15/00, C 12 P 21/02**

(22) Date of filing: **16.07.86**

(30) Priority: **18.07.85 US 756355**

(71) Applicant: **LUBRIZOL GENETICS INC., 3375 Mitchell Lane, Boulder Colorado 80301-2244 (US)**

(43) Date of publication of application: **21.01.87 Bulletin 87/4**

(72) Inventor: **Appelbaum, Edward R., 17 Dumont Circle, Madison Wisconsin 53711 (US)**
Inventor: **Adang, Michael J., 601 Valley Road, Madison Wisconsin 53714 (US)**

(84) Designated Contracting States: **AT BE CH DE FR GB IT LI LU NL SE**

(74) Representative: **Fisher, Adrian John et al, Carpmaels & Ransford 43 Bloomsbury Square, London WC1A 2RA (GB)**

(54) **Insecticidal rhizobiaceae cells.**

(57) Complete and partial Bacillus thuringiensis var. kurstaki HD-73 crystal protein protoxin genes were cloned into pRK290. E. coli cells harboring these recombinant plasmids produced protein that was toxic to Manduca sexta (tobacco hornworm) larvae. These plasmids were unstable in and inhibitory of the growth of Agrobacterium and Rhizobium cells; the presence of a complete protoxin gene being far more destabilizing and inhibitory than the presence of a partial protoxin gene. Rhizobium cells containing a partial protoxin gene formed root nodules in legume roots which were toxic to M. sexta larvae. Plasmids carrying complete protoxin genes were totally incompatable with root nodule formation; bacteroids in nodules formed by inoculation with stains containing the complete gene did not contain the gene-carrying plasmid and nodules were not toxic to insect larvae. The invention provides methods to produce insecticidal protein by constructing partial protoxin genes followed by expression of the genes in bacteroids and Rhizobiaceae cells. Useful plasmids and cells are also provided.

## INSECTICIDAL RHIZOBIACEAE CELLS

### FIELD

The present invention is in the fields of genetic engineering and bacterial bio-affecting compositions, especially those derived from the genus Bacillus.

### BACKGROUND

The following are publications disclosing background information related to the present invention: H. E. Schnepf et al. (1985) J. Biol. Chem. 260:6264-6272, and H. E. Schnepf and H. R. Whiteley (1985) J. Biol. Chem. 260:6273-6280, disclosed the DNA sequence of the HD-1 gene and reported that the HD-1 toxin moiety resides in the amino-proximal 68 kD peptide, respectively. M. J. Adang, U.S. Patent application ser. no. 617,321, discloses the complete sequence of a B. thuringiensis var. kurstaki HD-73 crystal protein gene and teaches toxicity of E. coli cells expressing partial and complete HD-73 protoxin genes and the derivation of pBt73-16, pBt73-10, and pBt73-3(Ava). *

### Chemistry

Bacillus thuringiensis, a species of bacteria closely related to B. cereus, forms a proteinacious crystalline inclusion during sporulation. This crystal is parasporal, forming within the cell at the end opposite from the developing spore. The crystal protein, often referred to as the δ-endotoxin, has two forms: a nontoxic protoxin of approximate molecular weight (MW) of 130 kilodaltons (kD), and a toxin having an approx. MW of 68 kD. The crystal contains the protoxin protein which is activated in the gut of larvae of a number of insect species. During activation, the protoxin is cleaved,the toxic moiety residing in an amino-proximal 58-68 kD polypeptide. In vivo, the crystal is activated by being solubilized and converted to toxic form by the alkalinity and proteases of the insect gut. The crystal protein may be activated in vitro by solu-

* See also Adang, M.J. et al. "Characterized full length and truncated plasmid clones of the crystal protein of Bacillus thuringiensis subsp. Kurs aki HD-73 and their toxicity to Manduca sexta", Gene (1985) 36 : 289-300.

bilization followed by the action of a protease such as trypsin (R. M. Faust et al. (1974) J. Invertebr. Pathol. 24:365-373). Y. Nagamatsu et al. (1984) Agric. Biol. Chem. 48:611-619, report that tha amino-terminus of the toxic, trypsin-resistant peptide begins at amino acid 29 of the translation product; the resultant peptide is about 58 kdal. in size. H. E. Schnepf and H. R. Whitely (1985) J. Biol. Chem. 260:6273-6280, report that in HD-1 deletions to amino acid residue 50 from the amino-terminus or to residue 603 from the carboxy-terminus abolish toxicity while deletions 10 and 645 residues, respectively, from the amino and carboxy termini do not. Activation of the protoxin has been reviewed by H. E. Huber and P. Lüthy (1981) in Pathogenesis of Invertebrate Microbiol. Diseases, ed.: E. W. Davidson, pp. 209-234.

## Toxicology

B. thuringiensis and its crystalline endotoxin are useful because the crystal protein is an insecticidal protein known to be poisonous to the larvae of over a hundred of insect species, most commonly those from the orders Lepidoptera and Diptera. Many of these species are economically important pests. B. thuringiensis varieties can differ in host range (R. M. Faust et al. (1982) in Genetic Engineering in the Plant Sciences, ed. N. J. Panapolous, pp. 225-254). The efficacy and safety of the endotoxin have been reviewed by Faust et al., supra. Other useful reviews include those by P. G. Fast (1981) in Microbial Control of Pests and Plant Diseases, 1970-1980, ed.: H. D. Burges, pp. 223-248, and H. E. Huber and P. Lüthy (1981) in Pathogenesis of Invertebrate Microbial Diseases, ed.: E. W. Davidson, pp. 209-234.

## Molecular Biology

The crystal protein gene usually can be found on one of several large plasmids that have been found in Bacillus thuringiensis, though in some strains it may be located on the chromosome (J. W. Kronstad et al. (1983) J. Bacteriol. 154:419-428; J. M. Gonzalez Jr. et al. (1981) Plasmid 5:351-365). Crystal protein genes have been cloned into plasmids that can grow in E. coli by several laboratories (see below). E. coli strains expressing either complete or partial protoxin genes have not been noted to be inhibited in bacterial growth.

Whiteley's group (H. R. Whitely et al. (1982) in Molecular Cloning and Gene Regulation in Bacilli, eds.: A. T. Ganesan et al., pp. 131-144, H. E. Schnepf and H. R. Whiteley (1981) Proc. Natl. Acad. Sci. USA 78:2893-2897, and European Pat. application 63,949) reported cloning DNAs including the protoxin genes from B. thuringiensis var. kurstaki strains HD-1-Dipel and HD-73. The HD-1-Dipel gene was transcribed, probably from its own promoter, and translated in E. coli. Whiteley et al., supra, reported a construction deleting the 3'-end of the HD-1 protoxin encoding sequences. The deletion included sequences which encode part of the mature toxin. Transcriptional and translational start sites were determined by nucleic acid sequencing (H. C. Wong et al. (1983) J. Biol. Chem. 258:1960-1967). Wong et al. localized the HD-1 crystal protein gene by transposon mutagenesis, noting that transposon insertion in the 3'-end of the gene could result in production in E. coli of 68 kD peptides, but not reporting any insecticidal activity to be associated with extracts of strains that did not produce 130 kD protoxin. The HD-1 gene has been completely sequenced (H. E. Schnepf et al. (1985) J. Biol. Chem. 260:6264-6272). The amino-proximal 55% of the protoxin is sufficient for toxicity and the amino-terminal 10 amino acids residues are not required for toxicity (H. E. Schnepf and H. R. Whiteley (1985) J. Biol. Chem. 260:6273-6280). Peptides lacking the amino-terminal 50 amino acid residues were not toxic.

A. Klier et al. (1982) EMBO J. 1:791-799, and G. A. Held et al. (1982) Proc. Natl. Acad. Sci. USA 77:6065-6069, have reported cloning of crystal protein genes from B. thuringiensis strain berliner 1715 and var. kurstaki, respectively. Fragments carrying the crystal protein gene were ligated into the vector pHV33 (G. Rapoport et al. (1979) Mol. Gen. Genet. 176:239-245), which can replicate in both E. coli and Bacillus, but is not known to be maintained in Rhizobiaceae cells. In both E. coli and sporulating B. subtilis, the pHV33-based clone directed the synthesis of 130 kD complete protoxin.

M. J. Adang, U.S. Pat. app. ser. no. 617,321, discloses the complete sequence of a B. thuringiensis var. kurstaki HD-73 crystal protein gene

0209370

and teaches toxicity of <u>E</u>. <u>coli</u> cells expressing partial and complete HD-73 protoxin genes derived, and the derivation of pBt73-16, pBt73-10, and pBt73-3(Ava).

SUMMARY

In pursuance of goals detailed below, the present invention provides DNA plasmids carrying partial protoxin genes, a partial protoxin being a polypeptide comprising part of the amino acid sequence of naturally-occurring toxin and often other amino acid sequences but lacking some of the naturally-occurring protoxin amino acid sequences. These genes are expressible in bacterial cells of the family Rhizobiaceae, which includes the genera <u>Agrobacterium</u> and <u>Rhizobium</u>. Unexpectedly, although complete protoxins produced by these genes as disclosed herein have proven growth-inhibitory to Rhizobiaceae cells when expressed in such cells, it was discovered that partial protoxins are significantly less growth-inhibitory. Methods useful toward construction of partial protoxin genes and expression of partial protoxin proteins are also provided.

The <u>Bacillus thuringiensis</u> crystal protein is useful as an insecticide because it is highly specific in its toxicity, being totally nontoxic against most nontarget organisms. As protoxin crystals must be ingested for toxicity, the crystal must be located where they will be eaten by larvae. Therefore, it is an object of the present invention to provide bacteria capable of colonizing a plant or forming a symbiosis with a plant. As expression of a complete protoxin gene within a Rhizobiaceae cell severely slows growth of that cell and severely destablizes plasmids maintaining that gene within that cell, it is an additional object to provide structural genes derived from complete protoxin genes which are compatable with Rhizobiaceae cell growth while being toxic to insect larvae.

BRIEF DESCRIPTION OF THE DRAWINGS

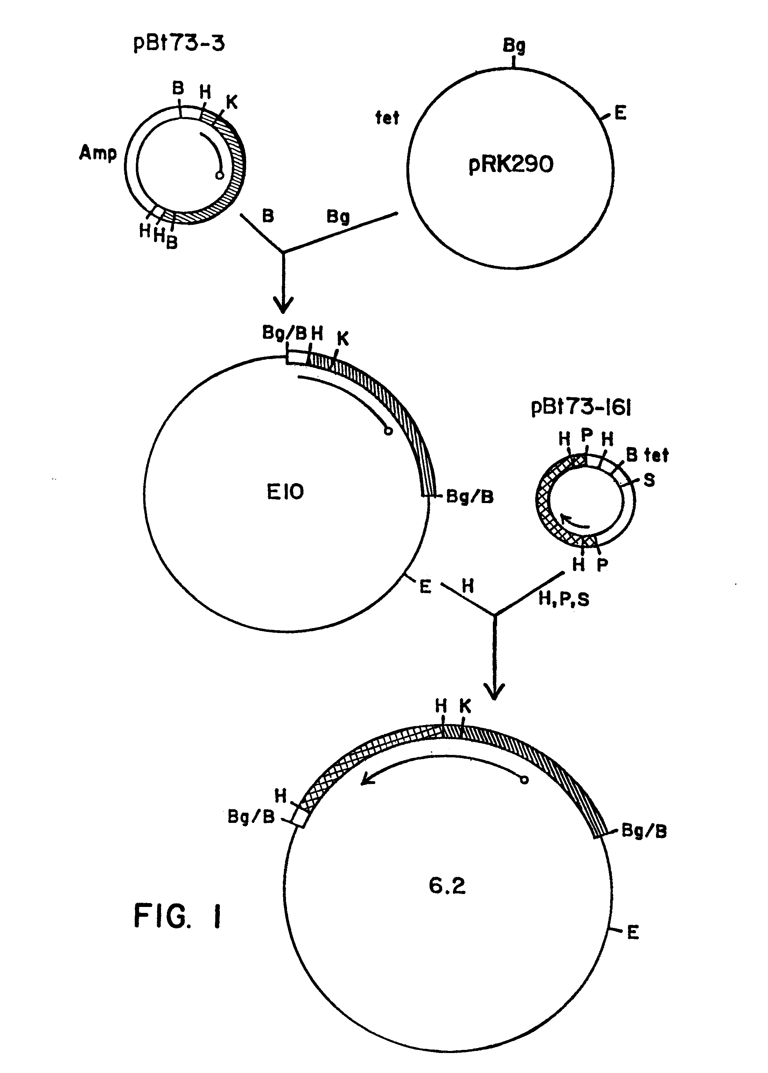

Figure 1 diagrams schematically and not necessarily to scale DNA manipulations described in Example 2. Amp and tet respectively refer to functional ampicillin and tetracycline resistance genes. The unattached
curved lines with the plasmid-representing circles indicate locations of
protoxin-encoding sequences, a small circle and an arrow head respectively
denoting the amino terminus (5'-end) and the carboxy terminus (3'-end).
Restriction enzymes and sites are indicated as follows: B, BamHI;
H, HindIII; K, KpnI; Bg, BglII; E, EcoRI; P, PstI; and S, SalI.
Plasmid sequences are represented as follows: thin lines are derived from
pRK290; open boxes are derived from pBR322; and various hatched boxes
are derived from B. thuringiensis.

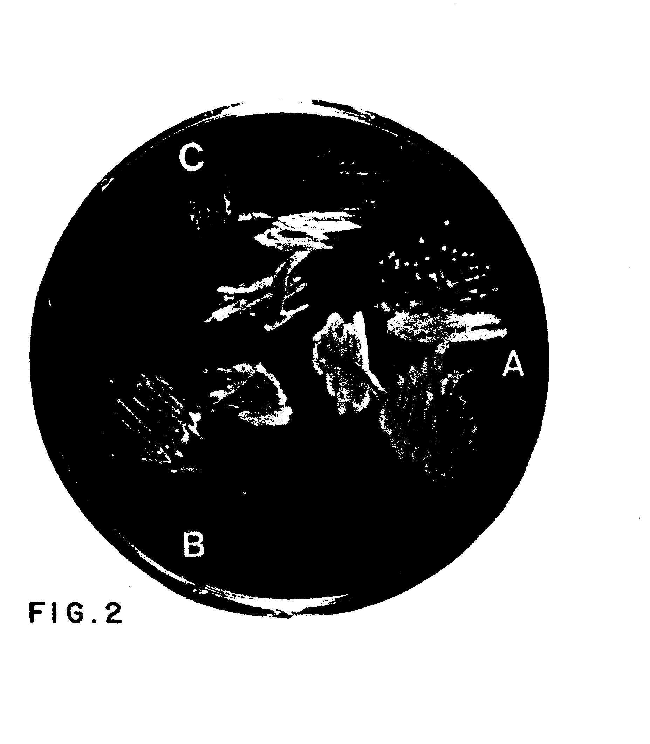

Figure 2 is a photograph of colonies of USDA191 $str^r$ derivatives
harboring the plasmids pRK290, E10, and 6.2, as indicated by A, B, and C,
respectively.

## DETAILED DESCRIPTION OF THE INVENTION

The following terms are defined in order to remove ambiguities to the
intent or scope of their usage in the Specification and Claims.

Complete protoxin, or protoxin, refers herein to protein encoded by a
B. thuringiensis crystal protein gene. In the variety kurstaki, the
complete protoxin has an approximate molecular weight of 130,000 Daltons.

Complete toxin, or toxin, refers herein to an insecticidal protein
derived from a crystal protein, in particular, that part of the protoxin
that is refractory towards processes, such as proteolytic digestion, that
activate protoxin in nature. In the variety kurstaki, the complete toxin
has an approximate molecular weight of 68,000 Daltons and is lacking the
carboxy-terminal half of the protoxin.

Partial protoxin refers herein to a protein having part of the amino
acid sequence of protoxin and lacking part of the amino acid sequence of
the carboxy-terminus of the protoxin but not the carboxy-terminus of the
toxin. Modifications of protoxin amino acid sequence, including a deletion at the amino-terminus of the toxin, may or may not be present. The
partial protoxin may have at its carboxy-terminus an amino acid sequence
not present in the complete protoxin. In other words, a structural gene
open reading frame encoding partial protoxin may be lacking sequences

-5-

encoding the carboxy-terminus of the protoxin but not sequences encoding the carboxy-terminus of the toxin, and may include sequences coding for additional amino acids not present in the complete protoxin. The primary requirement of a partial protoxin is that it lack amino acid sequences present in the complete protoxin and that it be toxic to some insect larvae. Bioassays of putative partial protoxins are well understood by those of ordinary skill in the arts of insect physiology (e.g. see Example 3.5).

Complete protoxin gene, partial protoxin gene, and toxin gene refers herein to expressible structural genes encoding the indicated proteins, each structural gene having at its 5'-end a 5'...ATG...3' translational start signal and at its 3'-end a translational stop signal (TAG, TGA, or TAA). As is well understood in the art, the start and stop signals must be in the same reading frames, i.e. in the same phase, when the mRNA encoding a protein is translated, as translational stop codons that are not in frame are ignored by the translational machinery and are functionally nonexistant. Modifications of the genetic structure are not excluded as long as the designated protein is encoded by the transcript. Removal of sequences from complete protoxin genes to form partial protoxin genes is well understood by those of ordinary skill in the arts of recombinant DNA manipulations and DNA sequence analysis, and has been exemplified herein and in publications reviewed in the background.

Underlying the present invention are two surprising discoveries: 1) that expression of a complete protoxin gene, which is compatible with growth of gram-negative bacteria such as E. coli is not fully compatible with the growth of Rhizobium or Agrobacterium cells; and 2) that removal of the 3'-end of the protoxin gene, thereby forming a partial protoxin, makes protoxin gene expression more compatible with cell growth. We have also discovered that partial protoxin genes are expressed in Rhizobiaceae cells without having been placed behind a heterologous promoter, i.e. they are expressed under control of a Bacillus promoter. An additional surprising discovery is that a partial protoxin gene is expressed under control of a Bacillus promoter in Rhizobium bacteroids within root nodules; many rhizobial genes are expressed only in bacteriods or only in free-living bacteria. Also the presence within a root nodule cell of bacteroids containing partial protoxin and complete toxin does not affect the Rhizobium-plant symbiosis and is compatable with root nodule cell function. Root nodules having partial protoxin-containing bacteroids are toxic to insect larvae.

Production of an insecticidal protein in a Rhizobiaceae cell or Rhizobium bacteroid by means of expression of a partial protoxin gene combines specific teachings of the present disclosure with a variety of techniques and expedients known in the art. In most instances, alternative expedients exist for each stage of the overall process. The choice of expedients depends on variables such as the choice of B. thuringiensis strain and protoxin gene starting materials, means for and particulars of premature translational termination, vector carrying the artificial partial protoxin gene, promoters to drive partial protoxin gene expression, and Rhizobiaceae species and strains into which the partial protoxin gene/promoter combination is transformed and expressed. Many variants are possible for intermediates and intermediate steps, such as source and recipient organisms, vector, and DNA manipulation strategy.

In the practice of this invention, one ordinarily first obtains a recombinant DNA molecule carrying either a complete protoxin gene or a fragment of a protoxin gene. The means for constructing such recombinant DNA molecules are well known in the art. If the desired protoxin is carried by a Bacillus plasmid, one may prepare DNA enriched for the gene by first isolating that plasmid, as has been exemplified herein. Alternatively, one may make a collection recombinant DNA-containing strains from total B. thuringiensis DNA that is statistically likely to have at least one representative of a protoxin gene (i.e. a genomic clone library). The Bacillus DNA may be digested to completion with a restriction endonuclease that cleaves DNA rarely (a six-base-cutter, e.g. HindIII or PstI, averages one site in about 4 kbp) or may be digested incompletely (i.e. partial digestion) with an enzyme that cleaves often (a four-base-cutter, e.g. Sau3AI averages one site in about 0.25 kbp), adjusting digestion conditions so the cloned DNA fragments are large enough to be likely to contain a complete protoxin gene. The Bacillus DNA is then ligated into a vector. Commonly the vector is one that can be maintained in E. coli, though vectors maintainable in Bacillus species are also useful. The Bacillus DNA/vector combinations are then transformed into appropriate host cells. After a collection of candidates are created, a strain containing a protoxin gene/vector combination may be identified using any of a number expedients known to the art. One can grow candidates on nitrocellulose membrane filters, lyse the cells, fix the released DNA to the filters, and identify colonies containing protoxin DNA by hybridization. The hybridization probe can be derived from sources including a different cloned cross-hybridizing protoxin gene, sporulation-stage specific B. thuringiensis RNA, or a synthetic nucleic acid having a protoxin sequence deduced from the protoxin amino acid sequence. If the protoxin gene is expressed in its host, screening using bioassays for insecticidal activity or using immunological methods is possible. Immunological methods include various immunoassays (e.g. radioimmunoassays and enzyme-linked immunoassays) and a method analogous to the probing of nitrocellulose-bound, electrophoretically resolved DNA ("western blots"). Colonies grown on nitrocellulose filters may be lysed, protein bound to the filters, and colonies containing protoxin protein identified using enzyme- or radioisotope-labeled antibodies.

The construction of recombinant DNA molecules containing complete protoxin genes, partial protoxin genes, and incomplete toxin genes can become inextricably tied to each other. While trying to isolate a complete protoxin gene, one may find gene segments deleted for their 3'-sequences. An extreme case of 3'-deletion is when sequences encoding the carboxy-terminus of the toxin are missing from the initially cloned gene fragment, resulting in lack of insecticidal activity in the expressed polypeptide. Similar events can lead to isolation of gene fragments lacking 5'-sequences. Conversely, should one intend to construct a partial protoxin gene, initially a complete protoxin gene may fortuitously be isolated. The isolation of missing gene fragments and their use in reconstruction of larger partial genes and complete genes is well understood in the art of recombinant DNA manipulations and is exemplified herein. Generally, one uses the gene fragment one already has to make a probe that is then used to look for flanking sequences that overlap with the probe. Libraries made using partial restriction enzyme digestion conditions can be screened directly for Bacillus DNA fragments overlapping with the probe. Libraries made using complete restriction enzyme digestion must have been made using a different enzyme than was used to make the probe-supplying plasmids. As is understood in the art, it is advantageous to map flanking restriction sites by means of Southern blots before constructing a second library. It is also advantageous to sequence or otherwise characterize the overlaps so as to be sure the two fragments are derived from the same gene, and to sequence the suture between the two fragments so as to be sure that the fusion has been accomplished as planned and that the open reading frames has been preserved, e.g. that no frameshift mutations have been introduced.

The carboxy-proximal half of the crystal protein protoxin, encoded by the 3'-half of the protoxin gene, is not necessary for toxicity. A variety of protoxin gene products missing the natural carboxy-terminus (i.e. partial protoxin gene products) are processed in vivo in Rhizobiaceae cells to a polypeptide essentially indistinguishable from in vivo or in vitro proteolytically-derived toxin. This last aspect constrains the sequence of the partial protoxin gene; naturally-occurring complete protoxin gene sequences are removed from the 3'-end of the complete protein gene. By definition, a coding sequence is terminated at its 3'-end by a translational stop signal. Removal of a 3'-end sequence entails translational termination at a new site and, as the stop signal is approached, may entail departure from the naturally-encoded protoxin amino acid sequence. Coding sequences can be removed in several ways.

The native stop signal need not be physically deleted; it need only be made inaccessible to ribosomes translating a protoxin-encoding mRNA transcript. One means for making the native stop inaccessible is by introduction of a frameshift mutation, usually an insertion or deletion of one or two base pairs, 5'-to the native translational stop site, thereby shifting the native stop out of the reading frames of the toxin and shifting another TAA, TAG, or TGA sequence into the toxin's reading frame. Another means for making the native stop site inaccessible is by substitution or insertion of one to three base pairs, thereby directly creating a stop signal 5'-to the native stop at that site. As is well understood in the art, substitutions and frameshift mutations can be introduced by a number of methods, including oligonucleotide-directed, site-specific mutagenesis. Frameshift mutations may often be created by cleaving DNA with a sticky-end-generating restriction enzyme followed by converting the sticky-ends to blunt-ends and religation.

A number of embodiments involve deleting protoxin sequences which do not encode any part of the complete toxin,i. e. are nonessential for toxicity, from the 3'-half of the protoxin gene. If the deletion is flanked on either side by protoxin gene sequences, the deletion may introduce a frameshift leading to utilization of a new stop codon. If the deletion preserves the reading frames, it will lead to utilization of the naturally used stop codon while deleting part of the nontoxin protoxin gene sequence. Should the deletion remove the 3'-end of the protoxin structural gene, the open reading frame defined by the toxin will run into nonprotoxin DNA sequences and will eventually terminate in a stop codon in that reading frame. Nonprotoxin <u>Bacillus</u> sequences, vector DNA, and synthetic oligonucleotides are all examples of nonprotoxin DNAs that may encode a partial protoxin stop codon.

3'-sequences may also be removed by insertion of DNA into the 3'-half of a complete protoxin gene. Transposons provide a convenient means for interrupting the 3'-half of a complete protoxin gene. Alternatively, DNA may be ligated into a restriction site. Translational termination occurs in non-<u>Bacillus</u> DNA at a stop codon, as described above for deletions. Similarly, substitutions (which may be thought of as a deletion followed by an insertion) may be used to remove the 3'-end of a complete protoxin gene.

As one of the goals of this invention is to express the partial protoxin gene in a Rhizobiaceae cell, the artificially constructed partial protoxin gene must be under control of a promoter capable of directing transcription in the desired Rhizobiaceae cell type, a consideration well understood in the art. Generally, one uses the recombinant DNA techniques to place the structural gene and a promoter, the latter being known to drive transcription in the cell in which expression is desired, in such position and orientation with respect to one another that the structural gene is expressed after introduction into recipient cell. In the preferred embodiments a partial protoxin gene is under control of a promoter expressible in a Rhizobiaceae cell in the free-living or bacterial states. A special case is when during the isolation of the protoxin structural gene, a protoxin gene promoter is isolated along with the protoxin structural gene, the protoxin promoter being the promoter which in
B. thuringiensis drives the expression of the protoxin gene. As disclosed
herein, this promoter/protoxin gene combination, which may also be
referred to as a Bacillus-expressible complete protoxin gene, was discovered to drive expression, when inserted in pRK290, in Rhizobiaceae
cells and Rhizobium bacteriods of both complete and partial protoxin
genes. Placement of a known Rhizobium promoter in front of the protoxin
genes was not necessary. In Bacillus this HD-73 promoter region drives
protoxin gene transcription only during sporulation.

The promoter/partial protoxin structural gene combination is then
placed in a known vector suitable for maintenance in the desired
Rhizobiaceae cell type. The promoter/structural gene/vector combination
is then introduced by an appropriate technique known in the art into a
cell of that cell type or from which that cell type may be derived, and
partial protoxin expression may be detected as described above. The
present application exemplifies expression of a partial protoxin gene in
Rhizobium cells and bacteriods and Agrobacterium cells under control of a
promoter derived from the same Bacillus-expressible complete protoxin
gene. Expression of partial protoxin genes under control of Rhizobiaceae
promoters will be well understood by those of ordinary skill in the art.


EXAMPLES

The following Examples are presented for the purpose of illustrating
specific embodiments within the scope of the present invention without
limiting the scope, the scope being defined by the Claims. Numerous
variations will be readily apparent to those of ordinary skill in the art.

These Examples utilize many techniques well known and accessible to
those skilled in the arts of molecular biology; such methods are fully
described in one or more of the cited references if not described in
detail herein. Enzymes are obtained from commercial sources and are used
according to the vendor's recommendations or other variations known to the
art. Reagents, buffers, and culture conditions are also known to those in
the art. Reference works containing such standard techniques include the

following:  R. Wu, ed. (1979) Meth. Enzymol. 68; R. Wu et al., eds. (1983) Meth. Enzymol. 100 and 101; L. Grossman and K. Moldave, eds. (1980) Meth. Enzymol. 65; J. H. Miller (1972) Experiments in Molecular Genetics; R. Davis et al. (1980) Advanced Bacterial Genetics; R. F. Schleif and P. C. Wensick (1982) Practical Methods in Molecular Biology; T. Maniatis et al. (1982) Molecular Cloning; and R. L. Rodriguez and R. C. Tait (1983) Recombinant DNA Techniques.  Additionally, R. F. Lathe et al. (1983) Genet. Engin. 4:1-56, make useful comments on DNA manipulations.

Textual use of the name of a restriction endonuclease in isolation, e.g. "BclI", refers to use of that enzyme in an enzymatic digestion, except in a diagram where it can refer to the site of a sequence susceptable to action of that enzyme, e.g. a restriction site.  In the text, restriction sites are indicated by the additional use of the word "site", e.g. "BclI site".  The additional use of the word "fragment", e.g. "BclI fragment", indicates a linear double-stranded DNA molecule having ends generated by action of the named enzyme (e.g. a restriction fragment).  A phrase such as "BclI/SmaI fragment" indicates that the restriction fragment was generated by the action of two different enzymes, here BclI and SmaI, the two ends resulting from the action of different enzymes.  Note that the ends will have the characteristics of being "blunt" (fully base-paired) or "sticky" (i.e. having an unpaired single-stranded protuberance capable of base-pairing with a complementary single-stranded oligonucleotide) and that usually the sequence of a sticky-end will be determined by the specificity of the enzyme which produces it.

Strains parenthetically indicate a plasmid harbored within, e.g. E. coli HB101 (6.2).  All exemplified recombinant DNA constructions can be done using starting materials well known and widely available to the art or pBt73-10 and pBt73-16, which are respectively harbored by the deposited strains NRRL B-15612 and NRRL B-15759.

Example 1:  Molecular cloning of complete and partial protoxin genes and expression in E. coli

This Example teaches isolation of complete and partial protoxin genes and properties thereof.

1.1:  pBt73-10 and pBt73-3

The crystal protein gene in Bacillus thuringiensis var. kurstaki HD-73 is located on a 50 megadalton (MD) plasmid.  This plasmid DNA was digested with HindIII.  The resulting fragments were mixed with and ligated to HindIII-linearized pBR322 (F. Bolivar et al. (1978) Gene 2:95-113) and transformed into E. coli HB101.  Ampicillin-resistant tetracycline-sensitive transformants were screened by digesting isolated plasmid DNA with HindIII and choosing those clones with 6.7 kilobase pair (kbp) inserts.  Bioassays of strains harboring pBt73-3 or pBt73-10 demonstrated the presence of B. thuringiensis insecticidal protein.

Restriction enzyme analysis of pBt73-3 and pBt73-10 showed that the two plasmids had identical 6.7 kbp B. thuringiensis DNA fragments inserted into the pBR322 vector in opposite orientations; the protoxin coding sequence of pBt73-10 was parallel to the pBR322 ampicillin gene while the pBt73-3 protoxin was antiparallel.  pBt73-3 can be converted to pBt73-10 by digestion with HindIII, religation, and transformation into HB101 followed by appropriate selection and screening steps.  Immunodetection of electrophoretically separated peptides on protein blots and DNA sequencing showed that pBt73-10 and pBt73-3 each contained a partial protoxin gene.

1.2:  pBt73-161

DNA enriched for the 50 MD plasmid was digested to completion with PstI, mixed with and ligated to PstI-linearized pBR322, and transformed into HB101.  Tetracycline-resistant transformants were screened essentially as described by W. D. Benton and R. W. Davis (1977) Science 196:180-182, using a probe nick-translated from the 6.7 kbp HindIII insert of pBt73-10.  Plasmid DNAs isolated from strains which bound the probe were characterized by restriction enzyme analysis.  A strain was identified which harbored a plasmid, designated pBt73-161, containing the 3'-end of a crystal protein gene.

-13-

### 1.3:  pBt73-16

The 5'- and 3'-ends of the protoxin gene were then fused together at the unique HindIII site to form a complete protoxin gene.  pBt73-10 DNA was digested with BamHI, ligated to itself, and transformed into HB101. Plasmid DNAs from ampicillin-resistant transformants were characterized by restriction enzyme analysis and a strain was identified that harbored a plasmid, designated pBt73-10(Bam), having single BamHI and HindIII sites due to deletion of a small HindIII site-bearing BamHI fragment.  A 5 kbp HindIII fragment of pBt73-161, isolated by agarose gel electrophoresis, was mixed with and ligated to HindIII-digested, dephosphorylated pBt73-10(Bam) DNA.  After the ligation mixture was transformed into HB101, plasmid DNAs isolated from ampicillin-resistant, tetracycline-sensitive transformants were characterized by restriction enzyme analysis.  A transformant was identified that harbored a plasmid, designated pBt73-16, carrying a complete protoxin gene.

### 1.4:  pBt73-3(Ava)

Convenient AvaI restriction sites in clone pBt73-3 were used to remove a 3' segment of the protoxin gene.  pBt73-3 DNA was digested with AvaI, ligated to itself, and transformed into HB101.  Plasmid DNAs isolated from ampicillin-resistant transformants were characterized by restriction enzyme analysis and a colony harboring a plasmid, designated pBt73-3(Ava), was identified.

### 1.5:  Expression of complete and partial protoxin genes in E. coli

Plasmid pBt73-16 was shown by DNA sequencing to contain a 3537 bp complete protoxin gene encoding a 133,333 D polypeptide.  E. coli cells containing this plasmid were observed to synthesize a peptide of approximately 130 kD that was immunologically and electrophoretically indistinguishable from complete protoxin protein and contained a 68 kD peptide similarly indistinguishable from complete toxin.

pBt73-10 carries the 5' 2,825 bp of the HD-73 protoxin gene, encoding a partial protoxin peptide sequence of 106,340 D.  Translation should continue into pBR322 encoded sequence for an additional 78 bases, thereby resulting in synthesis of a peptide having a total molecular weight of approximately 106,560 D. Analyses on the protein produced by E. coli cells harboring pBt73-3 or pBt73-10 did not contain 130 kD complete protoxin. However, peptides of about 68 kD and about 104 kD were observed.

pBt73-3(Ava) encodes in 1836 bp an amino-terminal 68,591 D peptide of the protoxin gene along with 32 amino acids encoded by pBR322 for an expected translation product of about 72 kD. E. coli (pBt73-3(Ava)) extracts contained a peptide of approximately 68 kD.

The 68 kD peptides were not distinguished from each other or activated crystal protein toxin by any tests used by the time this application was filed. Immunological, electrophoretic, chromatographic, and biological tests were tried.

Example 2: Construction of wide host-range plasmids carrying complete and partial protoxin genes and transfer to Rhizobiaceae cells

This Example teaches construction of plasmids capable of being maintained in both Rhizobiaceae and E. coli cells, the transfer of such plasmids from E. coli to Rhizobiaceae cells, and the expression therein. Construction of E10 and 6.2 is diagrammed in Figure 1.

2.1     E10

The partial protoxin gene may be removed from pBt73-3 on a 6.7 kbp BamHI fragment having all of pBt73-3's Bacillus DNA except for a 0.38 kbp BamHI/HindIII segment 5'-from the protoxin structural gene, and having a 0.35 kbp pBR322-derived BamHI/HindIII segment. BamHI-digested pBt73-3 DNA was mixed with and ligated to BglII-digested, dephosphorylated pRK290 (pRK290 is a broad host range plasmid mobilizable by pRK2013; G. Ditta et al. (1980) Proc. Natl. Acad. Sci. USA 77:7347-7357; for an example of its use, see G. B. Ruvkun and F. M. Ausubel (1981) Nature 289:85-88). The ligation mixture was transformed into E. coli HB101 and a tetracycline-resistant transformant was identified which harbored a plasmid, designated E10, having the 3'-end of the partial protoxin gene distal to the pRK290 EcoRI site and proximal to the pRK290 tet gene.

2.2:    6.2

pBt73-161 was digested with HindIII, PstI, and SalI, the latter two enzymes being used to degrade the pBR322 vector, and crystal protein sequences being on a 5 kbp HindIII fragment.  The digested pBt73-161 DNA was mixed with and ligated to HindIII-linearized, dephosphorylated E10 DNA.  Alternatively, the 5 kbp HindIII fragment is isolated from pBt73-16 by digestion with HindIII and electrophoretic isolation or by digestion with HindIII, PstI, and SalI; the 5 kbp fragment is then mixed with and ligated to HindIII-linearized, dephosphorylated E10 DNA.  The ligation mixture was transformed into HB101 and a tetracycline-resistant transformant was identified which harbored a plasmid, designated 6.2, having a 5 kbp Bacillus DNA HindIII fragment carrying complete protoxin gene and a 0.35 kbp pBR322 HindIII/BamHI fragment 3'-to the protoxin., the Bacillus and pBR322 sequences being inserted into the pRK290 BglII site, the pBR322 sequence and the 3'-end of the protoxin gene being proximal to the pRK290 tet gene and distal to the pRK290 EcoRI site.

2.3    Transfer to Rhizobiaceae cells

Plasmids E10 and 6.2 were mobilized into Agrobacterium tumefaciens ATCC 15955 and Rhizobium japonicum USDA191 str$^r$ (USDA191 is a fast-growing Rhizobium strain, H. H. Keyser et al. (1982) Science 215:1631-1632) by introduction of pRK2013 (G. Ditta et al., supra) in a triparental mating, a method well known in the art (e.g., see Ruvkun and Ausubel, supra).

2.4    Biological Properties in Rhizobiaceae cells

Crystal Protein sequences were shown to be expressed in both USDA191 str$^r$ (6.2) by bioassays and by immunological detection of blotted proteins after electrophoretic separation, while a USDA191 str$^r$ (pRK290) control was not toxic to larvae and had no immunologically detectable toxin or protoxin sequences.

In liquid culture, USDA191 str$^r$ (E10) was observed to grow somewhat more slowly than the USDA191 str$^r$ (pRK290) control, while USDA191 str$^r$ (6.2) was observed to grow very much more slowly than the control.  The differences in growth rates were easily observed when these strains were grown on a solidified medium (Figure 2).  The strain harboring E10 (B of Fig. 2) for colonies somewhat smaller than the control (A of Fig. 2), while the strain harboring 6.2 (C of Fig. 2) formed very small colonies that were not visible until long after the partial protoxin gene-containing and control strains were observed.

The presence of expressible crystal protein gene sequences destabilized pRK290-based vectors. When grown in the absence of tetracycline selection in liquid culture in a rich medium, TY (5 g/l tryptone, 3 g/l yeast extract, 7 mM $CaCl_2$) 49 out of 50 isolates of a USDA191 $str^r$ (pRK290) culture were found to be tetracycline resistant, while that was true of only 10 out of 50 USDA191 $str^r$ (E10) derived isolates. The strains harboring pRK290, E10, and 6.2 were also inoculated onto soybeans; isolates from surface sterilized nodules harvested 18 and 24 days after inoculation were tested for tetracycline resistance. Using a USDA 191 $str^r$ (pRK290) inoculum, 12 of 12 and 16 of 16 nodules respectively from 18 and 24 day plants, yielded drug resistant isolates while using a USDA191 $str^r$ (E10) inoculum, 7 of 12 and 5 of 16 nodules respectively from 18 and 24 day nodules yielded drug resistant isolates. Two of 16 R. japonicum nodules from 18 day plants inoculated with USDA191 $str^r$ (6.2) yielded tetracycline-resistant isolates (24 day root nodules were not tested). Extracts of root nodules formed by inoculation with USDA191 $str^r$ (E10) were found to be toxic to M. sexta larvae.

These results indicated that expression of crystal protein sequences slowed growth of Rhizobiaceae cells and either destablized or selected for loss of plasmids carrying the sequences, the effect being more severe for complete protoxin sequences than for partial protoxin sequences. Expression of complete protoxin sequences was incompatible with root nodule formation while partial protoxin sequences were compatable with nodule formation and when expressed in root nodules resulted in formation of nodules toxic to insect larvae.

Plasmids pRK290, E10, and 6.2 were also transferred to Agrobacterium tumefaciens ATCC 15955. Growth and insecticial properties of Agrobacterium cells harboring these plasmids was similar to Rhizobium cells harboring the same plasmids, growth slowing and colony size and stability decreasing in the series pRK290, E10, and 6.2. Extracts of 15955 (E10) and 15955 (6.2) were toxic to insect larvae while an extract of 15955 (pRK290) was not.

Example 3: Experimental

This Example discloses material and methods used in Examples 1 and 2.

3.1: Bacterial strains

Bacillus thuringiensis var. kurstaki strain HD-73 (NRRL B-4488) was from the Bacillus Genetics Stock Collection. B. thuringiensis var. kurstaki HD-1 (NRRL B-3792) was isolated from Dipel (Abbott Laboratories). Eschericia coli strain HB101 (NRRL B-11371) (H. W. Boyer and D. Roulland-Dussoix (1969) J. Mol. Biol. 41:459-472 was used in all E. coli transformations. E. coli HB101 (p123/58-10) (p123/58-10 is referred to herein as pBt73-10) and E. coli HB101 (pBt73-16) are on deposit as NRRL B-15612 and NRRL B-15759, respectively, with Northern Regional Research Center, 1815 N. University St., Peoria, Illinois 61604 USA. A. tumefaciens 15955 is on deposit as ATCC 15955 with American Type Culture Collection, 12301 Paklawn Dr., Rockville, Maryland 20852 USA.

3.2: Preparation of plasmids

Both pBR322 and B. thuringiensis plasmid DNA was prepared by an alkaline lysis method (H. C. Birnboim and J. Doly (1979) Nucl. Acids Res. 7:1513-1523). Before cloning, B. thuringiensis plasmids were fractionated by centrifugation at 39,000 rpm in a Beckman SW40-1 rotor for 90 min. at 15°C through 5%-25% sucrose gradients containing 0.55 M NaCl, 0.005 M NaEDTA, and 0.05 M Tris-HCl, pH 8.0, and the fractions analyzed on 0.5% agarose gels. Linearized vector DNAs were usually dephosphorylated by incubation with bacterial alkaline phosphatase before being mixed with and ligated to a DNA intended for insertion into the vector.

3.3: Preparation of antisera to crystal protein

B. thuringiensis strains HD-1-Dipel and HD-73 were grown to sporulation in modified G medium (A. I. Aronson et al. (1971) J. Bacteriol. 106:1016-1025 and crystals were purified by three passes in Hypaque-76 (Winthrop) gradients (K. Meenakshi and K. Jayaraman (1979) Arch.

Microbiol. 120:9-14), washed with 1 M NaCl, deionized water, and lyophilized. Crystals were solubilized in cracking buffer 1% SDS (sodium dodecylsulfate), 2% 2-mercaptoethanol, 6 M urea, 0.1 M sodium phosphate pH 7.2 with 0.02% bromphenol blue by heating at 95°C for 5 min. Electrophoresis was performed by a modification of the procedure of U. K. Laemmli (1970) Nature 227:680-685, as described previously (M. J. Adang and L. K. Miller (1982) J. Virol. 44:782-793). Gels were stained for 5 min, and destained 1 hour in deionized water. The 130 kD band was excised, lyophilized, and ground to a powder in a Wigl-Bug Amalgamator (Crescent Manufacturing Company). Rabbits were subcutaneously injected with 50 ng crystal protein, suspended in complete Freund's adjuvant followed by two injections with 50 ng crystal protein each in incomplete adjuvant over a four-week period. Monoclonal antibodies prepared against HD-73 crystal protein gave results identical in interpretation to results obtained with polyclonal sera.

### 3.4: Immunodetection of blotted peptides

E. coli clones were grown overnight in L-broth, pelleted, and brought to a 100 times concentrated suspension with 10 mM NaCl, 10 mM Tris HCl pH 8.0, and 1 mM EDTA containing phenylmethylsulfonyl fluoride (PMSF, a protease inhibitor) to 200 ng/ml. The suspension was sonicated on ice and the extracts stored frozen. Electrophoresis of E. coli extracts was as described above and immunodetection of peptides on "western" blots was according to the procedures of H. Towbin et al. (1979) Proc. Natl. Acad. Sci. USA 76:4350-4354.

### 3.5: Insect bioassays

Insects were obtained from commercial sources and kept essentially as described by R. A. Bell and F. G. Joachim (1976) Ann. Entomol. Soc. Amer. 69:365-373, or R. T. Yamamoto (1969) J. Econ. Entomol. 62:1427-1431. Bioassays for insecticidal protein were done by feeding extracts to larvae of Manduca sexta essentially as described by J. H. Schesser et al. (1977) Appl. Environ. Microbiol. 33:878-880. E. coli extracts for bioassays did not have PMSF in the sonication buffer.

CLAIMS

We claim:

1. A method for producing an insecticidal protein, comprising the steps of:
   (a) transforming a Rhizobiaceae cell to contain a DNA molecule comprising a partial protoxin gene; and
   (b) growing a Rhizobiaceae cell containing a partial protoxin gene under conditions wherein the protein encoded therein is expressed;
   whereby an insecticidal protein is produced.

2. A method according to claim 1, wherein coding sequences are removed from a complete or a partial protoxin gene by deleting.

3. A method according to claim 2, wherein after removing 3' sequences, the partial protoxin gene comprises sequences encoding a complete toxin.

4. A method according to claim 1, wherein the partial protoxin gene expressed in step (b) is under control of a promoter, the promoter and the partial protoxin gene being derived from the same Bacillus-expressible complete protoxin gene.

5. A method according to claim 1, wherein the cell of step (a) or step (b) is of the genera Agrobacterium or Rhizobium.

6. A method according to claim 1, wherein the protoxin is derived from a protoxin in essentially the same as is produced by B. thuringiensis var. kurstaki HD-73.

7. A method according to claim 6, wherein the HD-73-derived coding sequences of the protoxin or derivative gene essentially consist of HD-73-derived coding sequences of pBt73-16, pBt73-3, pBt73-10, or pBt73-3(Ava).

8. A recombinant DNA molecule comprising a DNA capable of being maintained in a Rhizobiaceae cell and a partial protoxin gene.

9. A DNA according to claim 8, wherein the partial protoxin gene is obtained from B. thuringiensis var. kurstaki HD-73.

-20-

10. A DNA according to claim 9, wherein the partial protoxin gene essentially comprises the coding sequence of pBt73-3(Ava), pBt73-16, pBt73-10, or pBt73-3.

11. A DNA according to claim 8, wherein the partial protoxin gene comprises sequences encoding a complete toxin.

12. A DNA according to claim 8, wherein the promoter and the partial protoxin gene being derived from the same Bacillus-expressible complete protoxin gene.

13. A DNA according to claim 8, wherein the Rhizobiaceae-maintainable DNA molecule is a plasmid.

14. A DNA according to claim 13, wherein the vector Rhizobiaceae-maintainable plasmid is pRK290 or a derivative thereof.

15. A DNA according to claims 10 or 14, wherein the DNA is or is a derivative of E10 or 6.2.

16. A DNA molecule derived from the DNA of claim 8.

17. A Rhizobiaceae cell or Rhizobium bacteriod containing the DNA of claim 16.

18. A cell according to claim 17, wherein the cell is of the genera Agrobacterium or Rhizobium.

19. A DNA according to claim 16, wherein the DNA is contained by a Rhizobiaceae cell or Rhizobium bacteriod.

20. A DNA according to claim 19, wherein the Rhizobiaceae cell is of the genera Agrobacterium or Rhizobium.

FIG. I

FIG.2

0209370

European Patent Office

Application number:

0209370

86 305 457.3

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

NRRL    B-15612
NRRL    B-15759
ATCC       15955